# EUROPÄISCHE PATENTANMELDUNG

(11) **EP 4 745 113 A1**
(43) Veröffentlichungstag der Anmeldung: **20.05.2026**
(21) Anmeldenummer: 24212576.3
(22) Anmeldetag: 13.11.2024
(51) Int. Cl.: C07C 29/151, C07C 31/04, C01B 3/04, C25B 1/04, C25B 15/08

(54) **VERFAHREN UND VORRICHTUNG ZUR ERZEUGUNG VON METHANOL**

(71) Anmelder: Linde GmbH, 82049 Pullach (DE)
(72) Erfinder: Bohn, Jan-Peter, c/o Linde GmbH IP EMEA 82049 Pullach (DE); Korn, Wibke, c/o Linde GmbH IP EMEA 82049 Pullach (DE); Taube, Carsten, c/o Linde GmbH IP EMEA 82049 Pullach (DE)
(74) Vertreter: Fischer, Werner

(57) **Zusammenfassung**

Die Erfindung betrifft ein Verfahren sowie eine Anlage zur Erzeugung von Methanol (e), bei dem ein Wassereinsatz (g) durch Elektrolyse (120) zerlegt wird, um Wasserstoff (c) für eine Methanolsynthese (130) zu gewinnen, bei der ein Methanol und Wasser enthaltenden Syntheseprodukt entsteht, aus dem zum Erhalt des Methanolprodukts (e) Wasser durch Kondensation abgetrennt wird, wobei ein überwiegend aus Wasser bestehendes Kondensat (g) anfällt. Kennzeichnend hierbei ist, dass zumindest ein Teil des Kondensats (g) zurückgeführt und der Elektrolyse (120) als Einsatz zugeführt wird.

## Beschreibung

Die Erfindung betrifft ein Verfahren zur Erzeugung von Methanol, bei dem ein Wassereinsatz durch Elektrolyse zerlegt wird, um Wasserstoff für eine Methanolsynthese zu gewinnen, bei der ein Methanol und Wasser enthaltendes Syntheseprodukt entsteht, aus dem zum Erhalt eines Methanolprodukts Wasser durch Kondensation abgetrennt wird, wobei ein überwiegend aus Wasser bestehendes Kondensat anfällt.

Weiterhin betrifft die Erfindung eine Anlage zur Durchführung des erfindungsgemäßen Verfahrens.

### Stand der Technik

Zur Gewinnung von Wasserstoff kann die sog. Elektrolyse eingesetzt werden, bei der Wasser durch elektrische Energie in Sauerstoff und Wasserstoff aufgespalten bzw. umgesetzt wird. Hierbei wird auch von der Wasser-Elektrolyse gesprochen. Insbesondere kann die für die Elektrolyse benötigte elektrische Energie aus erneuerbaren Energiequellen gewonnen werden; bei dem erhaltenen Wasserstoff wird dann von sog. grünem Wasserstoff gesprochen. Der Wasserstoff kann zusammen mit Kohlendioxid und/oder Kohlenmonoxid zur Synthese von Methanol verwendet werden, wobei Wasser und weitere Nebenprodukte anfallen.

Vor diesem Hintergrund stellt sich die Aufgabe, die Wasser-Elektrolyse mit der nachfolgenden Umsetzung von Wasserstoff zu Methanol zu verbessern, insbesondere energieeffizienter und/oder wirtschaftlicher zu machen.

### Offenbarung der Erfindung

Diese Aufgabe wird durch ein Verfahren sowie eine Vorrichtung der gattungsgemäßen Art zur Erzeugung von Methanol mit den Merkmalen der unabhängigen

### Patentansprüche gelöst. Ausgestaltungen sind Gegenstand der abhängigen

Patentansprüche sowie der nachfolgenden Beschreibung.

### Vorteile der Erfindung

Die Elektrolyse dient typischerweise dem Erzeugen oder Gewinnen von Wasserstoff. Bei der Wasser-Elektrolyse wird Wasser in Wasserstoff und Sauerstoff umgesetzt (gespalten), d.h. neben Wasserstoff wird zugleich auch immer Sauerstoff gewonnen bzw. erzeugt. Zur Wasser-Elektrolyse eignet sich z.B. die sog. alkalische Wasser-Elektrolyse (AEL, "Alkaline Electrolysis") oder die sog. Protonen-Austausch-Membran-Elektrolyse (PEM-Elektrolyse, "Proton Exchange Membrane"-Elektrolyse). Die Grundlagen hierzu sind an sich bekannt, z.B. aus " Bessarabov et al: PEM electrolysis for hydrogen production. CRC Press."

Daneben gibt es auch sog. Festoxid-Elektrolyseurzellen (SOEC, "Solid Oxide Electrolysis Cell") und die Anionen-Austausch-Membran-Elektrolyse (AEM-Elektrolyse, "Anion Exchange Membrane"-Elektrolyse).

Insbesondere kann, wie schon erwähnt, die für die Elektrolyse benötigte elektrische Energie aus erneuerbaren Energiequellen gewonnen werden; bei dem erhaltenen Wasserstoff wird dann von grünem Wasserstoff gesprochen. Hier kommen, als erneuerbaren Energiequellen z.B. Sonne und Wind in Betracht.

Bei dem erfindungsgemäßen Verfahren wird ein in der Elektrolyse erhältlicher wasserstoffhaltiger Fluidstrom einer Methanolsynthese zugeführt. Dieser wasserstoffhaltige Fluidstrom kann insbesondere aus dem gesamten bei der Elektrolyse erhaltenen Wasserstoff bestehen oder aber zumindest einen Großteil dieses Wasserstoffs umfassen.

Für die Methanolsynthese wird neben Wasserstoff auch Kohlendioxid und/oder Kohlenmonoxid benötigt. Das bei der Methanolsynthese gebildete Syntheseprodukt (Methanolsyntheseprodukt) enthält vorwiegend Methanol sowie bis zu 40Gew.-% Wasser. Daneben können weitere Nebenprodukte wie höhere Alkohole, Ester, Ether, Ketone und Kohlenwasserstoffe (im Einzelnen: Ethanol, Methylformiat, Aceton, Dimethylether und Methan) vorliegen.

Zum Erhalt eines Methanolprodukts wird Wasser von dem Methanolsyntheseprodukt durch Kondensation abgetrennt, wobei ein überwiegend aus Wasser bestehendes, weitere Nebenprodukte enthaltendes Kondensat anfällt, von dem erfindungsgemäß zumindest ein Teil zurückgeführt und in der Elektrolyse eingesetzt wird. Vorzugsweise wird das gesamte Kondensat zurückgeführt.

Auf diese Weise wird das bei dem Methanolsynthese anfallende Wasser zumindest teilweise wiederverwendet und nicht, wie bisher üblich, einfach entsorgt. Dies steigert nicht nur die Effizienz des Verfahrens bzw. der Anlage, sondern ist auch umweltschonender.

Im Methanol-Direktsynthese-Verfahren, bei dem Wasserstoff allein mit Kohlendioxid umgesetzt wird, fällt im Vergleich zum herkömmlichen Methanolsynthese-Verfahren, bei dem neben Kohlendioxid auch Kohlenmonoxid eingesetzt wird, mehr Wasser als Nebenprodukt an. Insofern ist die Effizienz und die Umweltverträglichkeit bei Anwendung des vorgeschlagenen Vorgehens bei der Methanol-Direktsynthese besonders hoch. Der Bedarf an Frischwasser wird reduziert.

Insbesondere wird das aus dem Methanolsyntheseprodukt abgetrennte Kondensat, bevor es der Elektrolyse als Einsatz zugeführt wird, einer Reinigung unterzogen. Auf diese Weise können Verunreinigungen wie Alkohole und Ameisensäure, die in dem Kondensat enthalten und in bei der Elektrolyse, insbesondere in der Elektrolyse-Zelle, problematisch sind, entfernt werden, um so hinreichend reines Wasser für die Zerlegung in der Elektrolyse zu erhalten.

Die Reinigung des Kondensats umfasst insbesondere wenigstens einen der folgenden Reinigungsvorgänge: eine Kondensat-Dampf-Strippung, eine Temperaturwechseladsorption, eine Aktivkohlefilterung, einen fortgeschrittenen Oxidationsprozesses (engl. "Advances Oxidation Process", AOP), einen lonenaustausch, eine Umkehrosmose, eine Membranverdampfung (z.B. Fallfilmeverdampfung), eine Membrandestillation, einen mit einer physikalischen Membrantrennung gekoppelten biologischen Reinigungsprozess (Biomembranreaktor, MBR, MMBR, Moving BedBioifilter Reactor), eine Elektrodeionisation (EDI) oder eine Elektrodialyseumkehr (Electrodialysis reversal, EDR).

Je nach Bedarf und Situation können einer oder mehrere dieser Reinigungsvorgänge vorgesehen sein, ggf. auch in einer bestimmten Reihenfolge. Eine Reinigungseinrichtung muss hierfür entsprechend eingerichtet sein. Je nach Art der Elektrolyse, also je nachdem, ob es sich z.B. um eine PEM-, AEL, AEM- oder SOEC-Elektrolyse handelt, können auch unterschiedliche Anforderungen an die Reinheit des Wassers gestellt werden, was z.B. auch verschiedene Arten der Reinigung erfordern kann.

Auch kann es gewisse Grenzen der Entfernung von Unreinheiten, insbesondere des sog. gesamten organischen Kohlenstoffs (engl.: "Total Organic Carbon", TOC) geben. Stromaufwärts der Elektrolyse liegt die Grenze für das Entfernen des gesamten organischen Kohlenstoffs z.B. bei etwa 250 ppb.

In einer Ausführungsform wird bei der Elektrolyse anfallende Wärme bei der Reinigung, der das zurückgeführte Kondensat unterzogen wird, verwendet. Die bei der Elektrolyse anfallende Wärme würde ansonsten nicht genutzt, sodass durch die Verwendung dieser Wärme bei der Kondensatreinigung die Energieeffizient des Verfahrens gesteigert wird. Eine Möglichkeit, die bei der Elektrolyse anfallende Wärme zu verwenden, bietet sich z.B. bei einer Kondensat-Dampf-Strippung oder einer Membrandestillation.

In einer Ausführungsform wird auch ein wasserhaltiger Elektrolyse-Fluidstrom, der bei der Elektrolyse nicht umgesetztes Wasser enthält, dem Wassereinsatz für die Elektrolyse zugeführt. Dieser wasserhaltige Elektrolyse-Fluidstrom wird sinnvollerweise, bevor er dem Wassereinsatz zugeführt wird, einer Reinigung unterzogen. Bei dieser Reinigung werden insbesondere polare organische und anorganische Verbindungen und TOC-erhöhende Stoffe entfernt. Im Fall der PEM-Elektrolyse wird TOC beispielsweise aus Rohrleitungen, Polisherharzen und der Membran selbst freigesetzt. Polare Komponenten sind z.B. organische Verbindungen wie Ameisensäure und elektrolysespezifische Verunreinigungen wie HF-Spezies (Grenzwert ist hier 1,5 µS/cm). TOC-erhöhende Verbindungen sind typischerweise Alkohole und organische Säuren sowie organische Amine (Grenzwert ist hier: 250 ppb). Technisch kann diese Reinigung durch die Integration eines Polisher-Systems realisiert werden.

In einer Ausführungsform werden der zurückgeführte Teil des Kondensats und der wasserhaltige Elektrolyse-Fluidstrom vereint und einer gemeinsamen Reinigung zugeführt. Die hierfür verwendete Reinigungseinrichtung kann dann ggf. die ansonsten in getrennten Reinigungseinrichtung vorgesehenen Reinigungsvorgänge, sofern verschiedene vorliegen, vereinen.

In einer Ausführungsform wird der Wassereinsatz, bevor er der Elektrolyse zugeführt wird, einer Wassereinsatz-Reinigung unterzogen. Der zurückgeführte Teil des Kondensats kann dann dem Wassereinsatz zugeführt werden, bevor dieser der Wassereinsatz-Reinigung unterzogen wird. Dies ermöglicht es, den zurückgeführten Teil des Kondensats noch besser zu reinigen und/oder die separate Reinigung Kondensats ggf. etwas zu vereinfachen. Für den Fall einer gemeinsamen Reinigung des zurückgeführten Teils des Kondensats und des wasserhaltigen Elektrolyse-Fluidstroms können diese als vereinter, gereinigter Fluidstrom dem Wassereinsatz zugeführt werden, bevor dieser der Wassereinsatz-Reinigung unterzogen wird.

Vorstehend beschriebene und nachfolgend noch zu beschreibende Ausführungsformen für das Verfahren gelten entsprechend auch für die Anlage, wozu ggf. nötige Komponenten oder Einheiten vorzusehen sind, sowie umgekehrt.

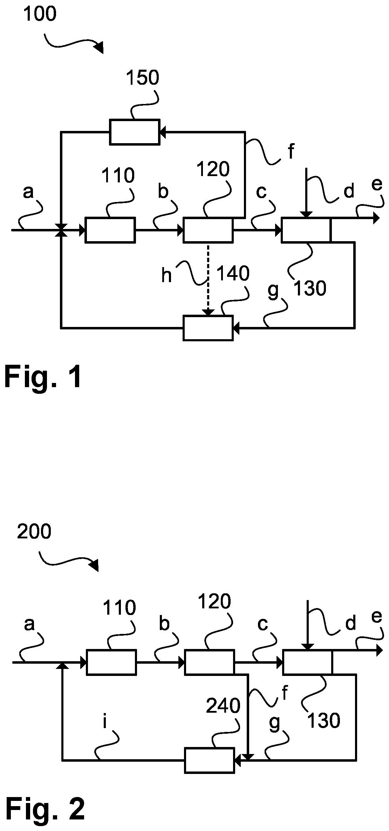

Die Erfindung wird nachfolgend unter Bezugnahme auf die beigefügten Figuren 1 und 2 näher erläutert, welche Anlagen gemäß zweier bevorzugter Ausführungsformen der vorliegenden Erfindung zeigen.

Kurze Beschreibung der Zeichnungen
Figur 1 zeigt schematisch eine erfindungsgemäße Methanolerzeugungsanlage in einer Ausführungsform.
Figur 2 zeigt schematisch eine erfindungsgemäße Methanolerzeugungsanlage in einer weiteren Ausführungsform.

### Ausführliche Beschreibung der Zeichnungen

In Figur 1 ist schematisch eine Methanolerzeugungsanlage 100 in einer Ausführungsform dargestellt, mit der eine bevorzugte Variante des erfindungsgemäßen Verfahrens durchführbar ist. Die Anlage und das Verfahren sollen nachfolgend überreifend beschrieben werden.

Die Methanolerzeugungsanlage 100 weist eine Elektrolyse-Einrichtung 120 und eine Methanolsynthese-Einrichtung 130 auf. In der Elektrolyse-Einrichtung 120, auch als Elektrolyseur bezeichnet, kann Wasser in Wasserstoff und Sauerstoff zerlegt werden. In der Methanolsynthese-Einrichtung 130 kann Wasserstoff mit Kohlendioxid und/oder Kohlenmonoxid in Methanol umgesetzt werden.

Als Elektrolyse-Einrichtung 120 kommen verschiedene Arten von Elektrolyse-Einrichtungen bzw. Elektrolyseuren infrage. Denkbar sind z.B. solche zur Durchführung einer PEM-, AEL, AEM- oder SOEC-Elektrolyse. Dabei handelt es sich insbesondere um eine Elektrolyse-Einrichtung im industriellen Maßstab, um Wasserstoff in industriellem Maßstab zu gewinnen. Die typische Leistung einer solchen Elektrolyse-Einrichtung liegt bei mehr als 10 MW oder auch mehr als 100 MW. Entsprechend handelt es sich bei der Methanolerzeugungsanlage 100 um eine solche im industriellen Maßstab; insbesondere kann mittels der Methanolsynthese-Einrichtung 130 der bei der Elektrolyse in der Elektrolyse-Einrichtung 120 erhaltene Wasserstoff vollständig umgesetzt werden.

Die Methanolerzeugungsanlage 100 weist Reinigungseinrichtungen 110, 140, 150 auf, die nachfolgend näher erläutert werden sollen.

Beim Betrieb der Methanolerzeugungsanlage 100 wird Frischwasser a, auch als Make-Up-Wasser bezeichnet, zunächst der Reinigungseinrichtung 110 zugeführt und dort initial gereinigt. Bei der Reinigungseinrichtung 110 kann es sich insbesondere um eine sog. Polishing Unit handeln, in der das Frischwasser a gereinigt wird. Das so gereinigte Frischwasser a wird dann als Teil eines Wassereinsatzes b der Elektrolyse-Einrichtung 120 zugeführt, um in Wasserstoff und Sauerstoff zerlegt zu werden.

Ein Wasserstoffstrom c, der zumindest einen Teil des bei der Elektrolyse erhaltenen Wasserstoffs umfasst, wird der Methanolsynthese-Einrichtung 130 für eine Methanolsynthese zugeführt. Neben dem Wasserstoff c wird der Methanolsynthese-Einrichtung 130 Kohlendioxid und/oder Kohlenmonoxid zugeführt, gemeinsam als Strom d bezeichnet. Ob Kohlendioxid oder Kohlenmonoxid oder beides zugeführt wird, hängt von der Art der Methanolsynthese-Einrichtung 130 bzw. des durchgeführten Art der Methanolsynthese ab.

Bei der Synthese wird ein Methanolsyntheseprodukt erhalten, das neben Methanol auch Wasser sowie weitere Nebenprodukte umfasst. In einer nicht dargestellten Trenneinrichtung wird im Syntheseprodukt enthaltenes Wasser abgetrennt, wobei Methanol e sowie ein weitgehend aus Wasser bestehendes, weitere Nebenprodukte aufweisendes Kondensat g erhalten werden. Während das Methanol e abgeführt und beispielsweise einer Speichereinrichtung (nicht dargestellt) zugeführt wird, wird das Kondensat g zurückgeführt und in der Reinigungseinrichtung 140 zu einem Wassereinsatz für die Elektrolyse-Einrichtung 120 aufbereitet.

Die Reinigungseinrichtung 140 kann z.B. Einrichtungen zur Kondensat-Dampf-Strippung, zur Temperaturwechseladsorption, zur Aktivkohlefilterung, zur Durchführung eines fortgeschrittenen Oxidationsprozess aufweisen. Auch können mehrere dieser Einrichtungen vorgesehen sein, die je nach Bedarf z.B. so angeordnet sind, dass die dabei durchgeführten Reinigungsvorgängen in einer bestimmten Reihenfolge durchlaufen werden.

Das auf diese Weise gereinigte Kondensat g wird nachfolgend dem Make-up-Wasser a zugeführt, mit dem es in die Reinigungseinrichtung 110 weitergeführt wird. Wenn die optionale Reinigungseinrichtung 110 nicht verwendet wird, kann das gereinigte Kondensat g dem Wassereinsatz b zugeführt werden. Auch wenn die Reinigungseinrichtung 110 verwendet wird, ist es möglich, dass das gereinigte Kondensat g dem Wassereinsatz b nach der Reinigungseinrichtung 110 zugeführt wird.

Bei der Elektrolyse 120 wird nur ein Teil des im Wassereinsatz b enthaltenen Wassers in Wasserstoff und Sauerstoff umgesetzt, so dass ein wasserhaltiger Elektrolyse-Fluidstrom f, der zumindest einen Teil des bei der Elektrolyse nicht umgesetzten Wassers enthält, ebenfalls dem Wassereinsatz b zugeführt werden kann. Der wasserhaltige Elektrolyse-Fluidstrom f wird sinnvolerweise, bevor er dem Wassereinsatz b bzw. dem Make-up-Wasser a zugeführt wird, in der Reinigungseinrichtung 150 einer Reinigung unterzogen.

In einer Ausführungsform kann bei der Elektrolyse 120 anfallende Wärme h bei der Reinigung des zurückgeführten Kondensats g eingesetzt werden. Die Wärme h kann hierzu der Reinigungseinrichtung 140 zugeführt werden und dort für die Betrieb einer oder mehrerer Einrichtungen wie vorstehend erwähnt verwendet werden. Falls zweckmäßig oder nötig, kann das Temperaturniveau des Wärmestroms h zusätzlich durch eine Wärmepumpe erhöht werden. Die Wärm h kann aber z.B. auch in der Reinigungseinrichtung 110 zur Erzeugung Wassereinsatzes b genutzt werden.

In Figur 2 ist schematisch eine Methanolerzeugungsanlage 200 in einer weiteren Ausführungsform dargestellt, mit der eine weitere bevorzugte Variante des erfindungsgemäßen Verfahrens durchführbar ist. Die Anlage und das Verfahren sollen nachfolgend überreifend beschrieben werden.

Die Methanolerzeugungsanlage 200 entspricht in weiten Teilen der Methanolerzeugungsanlage 100 gemäß Figur 1, sodass insofern auf die dortige Beschreibung verwiesen wird. Gleiche oder vergleichbare Einrichtungen oder Komponenten sowie Fluidströme sind mit gleichen Bezugszeichen versehen. Nachfolgend soll daher nur auf die Unterschiede eingegangen werden.

Die Methanolerzeugungsanlage 200 weist anstelle der Reinigungseinrichtungen 140 und 150 eine (gemeinsame) Reinigungseinrichtung 240 auf.

Der wasserhaltige Elektrolyse-Fluidstrom f, der zumindest einen Teil des bei der Elektrolyse 120 nicht umgesetzten Wassers enthält, wird hier dem zurückgeführten Kondensat g zugemischt, und zwar bevor letztes gereinigt wird. Es wird hier ein gemeinsamer oder vereinigter Fluidstrom i erhalten, der der Reinigungseinrichtung 240 zugeführt und dort einer Reinigung unterzogen wird.

Die Reinigungseinrichtung 240 kann z.B. alle Einrichtungen zur Reinigung enthalten, die gemäß der Ausführungsform der Figur 1 in den Reinigungseinrichtungen 140 und 150 getrennt vorgesehen sind. Denkbar ist auch, wenn zweckmäßig, dass gleiche oder vergleichbare Einrichtungen zur Reinigung, die gemäß der Ausführungsform der Figur 1 in den Reinigungseinrichtungen 140 und 150 getrennt vorgesehen sind, mit nur einer, ggf. größeren Einrichtung in der Reinigungseinrichtung 240 ersetzt sind.

Der auf diese Weise gereinigte Fluidstrom i wird dann dem Wasserstoffeinsatz b zugeführt. Insbesondere wird der gereinigte Fluidstrom i dem Wasserstoffeinsatz b vor der Reinigungseinrichtung 110 zugeführt. Wenn die optionale Reinigungseinrichtung 110 nicht verwendet wird, kann der Fluidstrom i einfach dem Wasserstoffeinsatz b zugeführt werden. Auch wenn die Reinigungseinrichtung 110 verwendet wird, ist es möglich, den Fluidstrom i dem Wasserstoffeinsatz b nach der Reinigungseinrichtung 110 zuzuführen.

## Patentansprüche

1. Verfahren zur Erzeugung von Methanol (e), bei dem ein Wassereinsatz (b) durch Elektrolyse (120) zerlegt wird, um Wasserstoff (c) für eine Methanolsynthese (130) zu gewinnen, bei der ein Methanol und Wasser enthaltenden Syntheseprodukt entsteht, aus dem zum Erhalt eines Methanolprodukts (e) Wasser durch Kondensation abgetrennt wird, wobei ein überwiegend aus Wasser bestehendes Kondensat (g) anfällt, **dadurch gekennzeichnet, dass** zumindest ein Teil des Kondensats (g) zurückgeführt und der Elektrolyse (120) als Einsatz zugeführt wird.

2. Verfahren nach Anspruch 1, wobei das zurückgeführte Kondensat (g) durch eine Reinigung (140) zum Einsatz für die Elektrolyse (120) aufbereitet wird.

3. Verfahren nach Anspruch 2, wobei die Reinigung (140), wenigstens einen der folgenden Reinigungsvorgänge umfasst:
- Kondensat-Dampf-Strippung,
- Temperaturwechseladsorption,
- Aktivkohlefilterung,
- fortgeschrittener Oxidationsprozess.
- Membranverdampfung
- Membrandestillation,
- lonenaustausch,
- Umkehrosmose,
- Elektrodeionisation
- Elektrodialyseumkehr,
- Biomembranreinigung.

4. Verfahren nach Anspruch 2 oder 3, wobei bei der Elektrolyse (120) anfallende Wärme (h) bei der Reinigung, des zurückgeführten Teils des Kondensats (g) eingesetzt wird.

5. Verfahren nach einem der vorstehenden Ansprüche, wobei ein wasserhaltiger Elektrolyse-Fluidstrom (f), der bei der Elektrolyse nicht umgesetztes Wasser enthält, dem Wassereinsatz (b) zugeführt wird.

6. Verfahren nach Anspruch 5, wobei der wasserhaltige Elektrolyse-Fluidstrom (f), bevor er dem Wassereinsatz (b) zugeführt wird, einer Reinigung unterzogen wird.

7. Verfahren nach Anspruch 6, wobei das zurückgeführte Kondensat (g) zusammen mit dem wasserhaltige Elektrolyse-Fluidstrom (f) einer gemeinsamen Reinigung zugeführt wird.

8. Verfahren nach einem der vorstehenden Ansprüche, **dadurch gekennzeichnet, dass** der Wassereinsatz (b) in einem Reinigungsschritt (110) erhalten wird, wobei das zurückgeführte Kondensat (g) dem Wassereinsatz stromauf- oder stromabwärts des Reinigungsschritts (110) zugeführt wird.

9. Methanolerzeugungsanlage (100, 200), mit einer Elektrolyse-Einrichtung (120), in der ein Wassereinsatz (g) durch Elektrolyse zerlegt werden kann, um Wasserstoff (c) zu gewinnen, einer Methanolsynthese-Einrichtung (130), der der Wasserstoff (c) zur Umsetzung in ein Methanol und Wasser enthaltendes Syntheseprodukt zuführbar ist, sowie einer Trenneinrichtung zur Zerlegung des Syntheseprodukts in ein Methanolprodukt (e) und ein überwiegend aus Wasser bestehendes Kondensat (g), **dadurch gekennzeichnet, dass** sie eine Rückführeinrichtung umfasst, über die zumindest ein Teil des Kondensats (g) zurückgeführt werden kann, um der Elektrolyse-Einrichtung (120) als Einsatz zugeführt zu werden.

10. Methanolerzeugungsanlage (100, 200) nach Anspruch 9, **dadurch gekennzeichnet, dass** die Rückführeinrichtung ein Reinigungseinrichtung (140, 240) umfasst, mit der das zurückgeführte Kondensat (g) zu einem Einsatz für die Elektrolyse-Einrichtung (120) aufbereitet werden kann.

11. Methanolerzeugungsanlage (100, 200) nach Anspruch 9 oder 10, wobei die Reinigungseinrichtung (140, 240) eingerichtet ist, wenigstens einen der folgenden Reinigungsvorgänge durchzuführen:
- eine Kondensat-Dampf-Strippung,
- eine Temperaturwechseladsorption,
- eine Aktivkohlefilterung,
- einen fortgeschrittenen Oxidationsprozess,
- eine Membranevaporation,
- eine Membrandestillation,
- einen lonenaustausch,
- eine Umkehrosmose,
- eine Elektrodeionisation,
- eine Elektrodialyseumkehr
- einen Biomembranreinigung.
